# FASCICULE DE BREVET EUROPEEN

(11) **EP 0 827 391 B1**
(45) Date de publication et mention de la délivrance du brevet: **14.08.2002**
(21) Numéro de dépôt: 97914389.8
(22) Date de dépôt: 13.03.1997
(51) Int. Cl.: A61K 7/06

(54) **COMPOSITION COSMETIQUE COMPRENANT UN POLYMERE ANIONIQUE ACRYLIQUE ET UNE SILICONE OXYALKYLENEE**
KOSMETISCHE ZUSAMMENSETZUNG, DIE EIN ANIONISCHES ACRYLPOLYMER UND EIN ALKOXYLIERTES SILICON ENTHÄLT
COSMETIC COMPOSITION INCLUDING AN ACRYLIC ANIONIC POLYMER AND AN OXYALKYLENE SILICONE

(30) Priorité: 14.03.1996 FR 9603233
(43) Date de publication de la demande: 11.03.1998
(73) Titulaire: L'OREAL, 75008 Paris (FR)
(72) Inventeur: DUPUIS, Christine, F-75018 Paris (FR)
(74) Mandataire: Andral, Christophe André Louis
(86) Numéro de dépôt international: FR9700450
(87) Numéro de publication internationale: WO9733554

(56) Documents cités:
- EP-A- 0 260 641
- US-A- 5 344 643
- PATENT ABSTRACTS OF JAPAN vol. 13, no. 427 (C-639) [3775] , 22 Septembre 1989 & JP 01 163113 A (SHISEIDO), 27 Juin 1989,

## Description

La présente invention concerne des compositions cosmétiques contenant, dans un milieu cosmétiquement acceptable, au moins un polymère fixant anionique acrylique et au moins une silicone oxyalkylénée particulière.

L'invention a encore pour objet un procédé de traitement cosmétique des matières kératiniques, telles que les cheveux ou les cils, caractérisé en ce qu'il consiste à appliquer sur les matières kératiniques une composition cosmétique telle que définie précédemment, puis à effectuer éventuellement un rinçage à l'eau.

Les compositions de maintien ou de mise en forme des cheveux contenant dans leur formulation des polymères de coiffage (polymères fixants) présentent généralement l'inconvénient de rendre difficile le démêlage, le recoiffage ou le brossage des cheveux, en particulier pendant un brushing.

Certains polymères présentent en outre l'inconvénient de poudrer sur la chevelure (présence de pellicules blanches) lors du brossage ou du peignage après séchage du polymère.

L'association de dérivés siliconés avec des polymères fixants est connue dans des compositions cosmétiques pour le maintien et/ou la fixation de la coiffure. Il a été constaté que ces dérivés siliconés améliorent les propriétés de démêlage, de douceur et de brillance des cheveux traités à l'aide de ces compositions. Cependant, d'une part, les dérivés siliconés ne sont pas favorables aux propriétés coiffantes et/ou fixantes des compositions contenant des polymères fixants et, d'autre part, les propriétés de démêlage et de douceur ne sont pas encore satisfaisantes.

Le but de la présente invention est donc de proposer des compositions permettant de fixer et/ou de mettre en forme la coiffure, ces compositions devant avoir un bon pouvoir fixant, de bonnes propriétés de tenue dans le temps, apporter de bonnes propriétés de toucher et de démêlage. De plus, elles doivent former un dépôt invisible sur les cheveux et ne pas poudrer lors du brossage ou du peignage.

Il est par ailleurs connu de la demande de brevet EP 260 641, des compositions coiffantes particulières dans lesquelles on associe des polymères siliconés spécifiques (comprenant au plus 90 unités SiO) à des polymères coiffants dans des émulsions particulières comprenant au moins un tensioactif non ionique ayant une valeur de HLB suppérieure ou égale à 10 et un tensioactif ionique. Les exemples proposés expriment clairement que lorsque l'on s'écarte faiblement de cette formulation particulière sous fomre d'émulsion, ont diminue tant les propriétés fixantes que cosmétique de ces compositions.

Or, la demanderesse vient maintenant de découvrir que des compositions cosmétiques contenant, dans un milieu cosmétiquement acceptable, au moins un polymère anionique acrylique et au moins une silicone oxyalkylénée particulière permettaient de façon inattendue et surprenante d'obtenir les propriétés décrites ci-dessus, notamment une diminution du poudrage, un toucher plus naturel, et une bonne tenue de la coiffure dans le temps, et ce sans nécessiter l'emploi d'une composition sous forme d'émulsion avec des tensioactifs très particuliers.

La présente invention a donc pour objet une nouvelle composition cosmétique caractérisée en ce qu'elle comprend, dans un milieu cosmétiquement acceptable, au moins un polymère fixant anionique dérivé d'acide acrylique ou méthacrylique et au moins une silicone oxyalkylénée particulière décrite ci-dessous.

Par polymère fixant, on entend selon l'invention tout polymère ayant pour fonction principale de fixer temporairement la forme de la coiffure.

Le polymère fixant anionique dérivé d'acide acrylique ou méthacrylique utilisable selon la présente invention est choisi parmi les polymères suivants :
A) les homopolymères d'acide acrylique ou méthacrylique ou leurs sels et en particulier les produits vendus sous les dénominations VERSICOL E ou K par la société ALLIED COLLOID.
B) les copolymères d'au moins un acide acrylique et/ou méthacrylique avec au moins un monomère monoéthylénique tel que l'éthylène, le styrène, les esters vinyliques, les acrylamides ou méthacrylamides éventuellement N-substitués, les esters d'acide acrylique ou méthacrylique, les vinyllactames. Ces copolymères sont éventuellement réticulés. De tels polymères sont décrits en particulier dans le brevet français 1.222.944 et la demande allemande 2.330.956, les copolymères de ce type comportant dans leur chaîne un motif acrylamide éventuellement N-alkylé et/ou hydroxyalkylé tels que décrits notamment dans les demandes de brevets luxembourgeois 75370 et 75371 ou proposés sous la dénomination QUADRAMER par la Société AMERICAN CYANAMID.

Parmi ces polymères, on peut citer :
- les copolymères d'acide acrylique et de méthacrylate d'alkyle, linéaire ou ramifié, en C₁-C₄,
- les copolymères d'acide méthacrylique et d'acrylate d'alkyle, linéaire ou ramifié, en C₁-C₄, tels que les copolymères d'acide méthacrylique et d'acrylate d'éthyle comme le LUVIMER MAE vendu par la société BASF,
- les copolymères d'acide acrylique et d'acrylate d'alkyle, linéaire ou ramifié, en C₁-C₄, tels que par exemple les copolymères d'acide acrylique et d'acrylate d'éthyle comme l'ACRYSOL 33 proposé par ROHM & HAAS,
- les copolymères d'acide méthacrylique et de méthacrylate d'alkyle, linéaire ou ramifié, en C₁-C₄, tels que notamment les copolymères d'acide méthacrylique et de méthacrylate de méthyle,
- les copolymères d'acide (méth)acrylique, de (méth)acrylate d'alkyle, linéaire ou ramifié, en C₁-C₂₀ et de vinylpyrrolidone, tels que celui vendu par la société ISP sous la dénomination ACRYLIDONE LM, ceux vendus par la société BASF sous les dénominations LUVIFLEX VBM 35 et LUVIFLEX VBM 70 ou celui vendu sous la dénomination STEPANHOLD EXTRA par la société STEPAN,
- les copolymères acide (méth)acrylique/ acrylate d'éthyle/ acrylate de tertiobutyle tel que le produit vendu sous la dénomination LUVIMER 100 P par la société BASF,
- les copolymères acide (méth)acrylique/ acrylate d'alkyle/méthacrylate d'alkyle en particulier le copolymère acrylate d'éthyle / méthacrylate de méthyle / acide méthacrylique / acide acrylique tels que le produit vendu sous la dénomination AMERHOLD DR 25 par la société AMERCHOL,
- les copolymères d'acide (méth)acrylique et d'au moins un acrylamide éventuellement **N**-substitués vendus par exemple sous les dénominations RETEN 421, 423 ou 425 par la Société HERCULES ou sous les dénominations ULTRAHOLD par la société BASF,
- les copolymères d'acide (méth)acrylique et de styrène,
- les copolymères d'acide (méth)acrylique et de vinylpyrrolidone tels que celui vendu sous la dénomination ACRYLIDONE ACP 1001 par la société ISP.

Selon l'invention, les polymères fixants anioniques sont de préférence choisis parmi les copolymères acide acrylique / acrylate d'éthyle / N-tertiobutylacrylamide vendu sous la dénomination ULTRAHOLD STRONG par la société BASF, les copolymères d'acide méthacrylique et de méthacrylate de méthyle vendus sous la dénomination EUDRAGIT L par la société ROHM PHARMA, le copolymère d'acide méthacrylique et d'acrylate d'éthyle vendu sous la dénomination LUVIMER MAEX ou MAE par la société BASF, le copolymère de vinylpyrrolidone / acide acrylique / méthacrylate de lauryle vendu sous la dénomination ACRYLIDONE LM par la société ISP.

Les silicones oxyalkylénées sont choisies parmi les composés de formule générale (I): 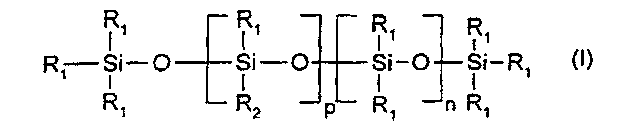 formule dans laquelle :
- R₁, identique ou diffèrent, représente un atome d'hydrogène, un radical alkyle linéaire ou ramifié en C₁-C₃₀ ou un radical phényle,
- R₂, identique ou différent, représente -(CₓH₂ₓ)-(OC₂H₄)ₐ-(OC₃H₆)_{b}-OR₃,
- R₃, identique ou différent, est choisi parmi un atome d'hydrogène, un radical alkyle, linéaire ou ramifié, ayant de 1 à 12 atomes de carbone, un radical acyle, linéaire ou ramifié ayant de 2 à 12 atomes de carbone,
- n varie de 100 à 1000,
- p varie de 1 à 8,
- a varie de 0 à 50,
- b varie de 0 à 50,
- a + b est supérieur ou égal à 1,
- x varie de 1 à 5,
le poids moléculaire moyen en nombre étant supérieur ou égal à 15.000 et de préférence compris entre 25.000 et 75.000.

De façon préférentielle, on utilise les silicones oxyalkylénées de formule générale (I) qui répondent à au moins une des, et de préférence à toutes les, conditions suivantes :
- R₁ désigne le radical méthyle;
- R₃ représente un atome d'hydrogène, un radical méthyle ou un radical acétyle et de préférence hydrogène;
- p varie de 2 à 6;
- a est compris entre 5 et 40 et de préférence entre 15 et 30;
- b est compris entre 5 et 40 et de préférence entre 15 et 30;
- x est égal à 2 ou 3;
- n varie de 200 à 600, de préférence de 300 à 500 et encore plus particulèrement de 200 à 500.

De telles silicones sont par exemple décrites dans le brevet US 4 311 695 qui est indu à titre de référence.

Les silicones les plus particulièrement préférées sont par exemple celles vendues en solution à 10% en poids dans une cyclométhicone (DOW CORNING 344) sous la dénomination commerciale FLUID DC 3225 C par la société DOW CORNING.

Le ou les polymères anioniques fixants sont généralement présents dans des concentrations comprises entre 0,1% et 20% en poids, et de préférence dans des concentrations comprises entre 1% et 10% en poids par rapport au poids total de la composition.

La ou les silicones oxyalkylénées peuvent être présentes dans des concentrations comprises entre 0,001% et 10% en poids, et de préférence dans des concentrations comprises entre 0,005% et 3% en poids par rapport au poids total de la composition.

Le milieu cosmétiquement acceptable comprend généralement des solvants compatibles avec le polymère fixant et la silicone oxyalkylénée. Ces solvants sont par exemple de l'eau, des alcools, de l'acétone, qui peuvent être utilisés seuls ou en mélange.

De préférence, on utilise des alcools en C₁-C₆. Parmi les alcools, on peut citer l'éthanol, l'isopropanol, les polyalcools tels que le diéthylèneglycol, les éthers de glycol, les monoalkyléthers de glycol, de diéthylèneglycol, de propylèneglycol ou de dipropylèneglycol. L'éthanol est particulièrement préféré.

Les compositions selon l'invention sont de préférence exemptes d'eau, c'est à dire qu'elle contiennent moins de 8% en poids d'eau par rapport au poids total de la composition et préférentiellement moins de 5%. Le séchage des compositions est ainsi plus rapide.

La composition de l'invention peut également contenir au moins un additif choisi parmi les épaississants, les esters d'acides gras, les esters d'acides gras et de glycérol, les silicones, les tensioactifs, les parfums, les conservateurs, les filtres solaires, les protéines, les vitamines, les polymères, les huiles végétales, animales, minérales ou synthétiques et tout autre additif classiquement utilisé dans le domaine cosmétique.

Ces additifs sont présents dans la composition selon l'invention dans des proportions pouvant aller de 0 à 40% en poids par rapport au poids total de la composition. La quantité précise de chaque additif est fonction de sa nature et est déterminée facilement par l'homme de l'art.

Bien entendu, l'homme de l'art veillera à choisir le ou les éventuels composés à ajouter à la composition selon l'invention de manière telle que les propriétés avantageuses attachées intrinsèquement à la composition conforme à l'invention ne soient pas, ou substantiellement pas, altérées par l'addition envisagée.

Les compositions selon l'invention peuvent se présenter sous forme de gels, de laits, de crèmes, de dispersions, d'émulsions, de lotions plus ou moins épaissies ou de mousses.

Les compositions selon l'invention sont utilisées plus particulièrement comme produits non-rincés notamment pour le maintien de la coiffure, la mise en forme ou le coiffage des cheveux.

Elles sont plus particulièrement des lotions de mise en plis, des lotions pour le brushing, des compositions de fixation (laques) et de coiffage. Les lotions peuvent être conditionnées sous diverses formes notamment dans des vaporisateurs, des flacons pompes ou dans des récipients aérosols afin d'assurer une application de la composition sous forme vaporisée ou sous forme de mousse. De telles formes de conditionnement sont indiquées, par exemple, lorsqu'on souhaite obtenir un spray, une laque ou une mousse pour la fixation ou le traitement des cheveux.

Lorsque la composition selon l'invention est conditionnée sous forme d'aérosol en vue d'obtenir une laque ou une mousse aérosol, elle comprend au moins un agent propulseur qui peut être choisi parmi les hydrocarbures volatils tels que le n-butane, le propane, l'isobutane, un hydrocarbure chloré et/ou fluoré et leurs mélanges. On peut également utiliser en tant qu'agent propulseur le gaz carbonique, le protoxyde d'azote, le diméthyléther, l'azote, l'air comprimé et leurs mélanges.

La concentration en propulseur est généralement comprise entre 10 et 90% en poids par rapport au poids total de la composition pressurisée et de préférence entre 15 et 70% en poids par rapport au poids total de la composition.

L'invention a encore pour objet un procédé de traitement cosmétique des matières kératiniques telles que les cheveux ou les cils consistant à appliquer sur celles-ci une composition telle que définie précédemment, puis à effectuer éventuellement un rinçage à l'eau.

L'invention va être maintenant plus complètement illustrée à l'aide des exemples suivants qui ne sauraient être considérés comme la limitant aux modes de réalisation décrits. Dans ce qui suit, MA signifie Matière Active.

### EXEMPLE 1

On a préparé une composition de spray fixant selon l'invention conditionnée dans un flacon-pompe de composition suivante :
- Terpolymère acide méthacrylique/ acrylate d'éthyle/ acrylate de tertiobutyle vendu sous la dénomination LUVIMER 100 P par la société BASF 7 g
- Amino-2 méthyl-2 propanol-1 qs neutralisation à 100% du polymère
- Silicone oxyalkylénée vendue à 10% de MA sous la dénomination FLUID DC 3225C par la société DOW CORNING 0,05 gMA
- Ethanol qsp 100 g

On a appliqué cette composition, après un brushing sur des cheveux lavés et séchés. Les cheveux sont maintenus en forme de façon naturelle et se démêlent aisément sans résidus poudreux. Les cheveux ont un toucher naturel agréable.

### EXEMPLE 2

On a préparé une composition (A) de spray fixant conditionnée dans un récipient aérosol de composition suivante :
- Terpolymère acide acrylique / acrylate d'éthyle / n-tertio butylacrylamide vendu sous la dénomination ULTRAHOLD STRONG par la société BASF 8 g
- Amino-2 méthyl-2 propanol-1 qs neutralisation à 100% du polymère
- Silicone oxyalkylénée vendue à 10% de MA sous la dénomination FLUID DC 3225C par la société DOW CORNING 0,06 gMA
- Ethanol qsp 100 g

Schéma de pressurisation :

| | |
|---|---|
| Composition ci-dessus : | 37 g |
| Diméthyléther | 43 g |
| Pentane | 20 g |

On introduit 37 g de la composition ci-dessus dans un récipient aérosol, on sertit une valve, puis on introduit le diméthyléther et le pentane. La composition présente les mêmes propriétés que celles de l'exemple 1.

On a préparé une composition (B) identique à la composition (A) dans laquelle on a remplacé le polymère acrylique selon l'invention (ULTRAHOLD STRONG) par la même quantité d'un polymère de type crotonique (terpolymère acide crotonique / acétate de vinyle / tertio-butylbenzoate de vinyle).

On a appliqué chacune de ces compositions sur des cheveux lavés et séchés. Un panel de 5 testeurs expérimentés a évalué les propriétés de chaque composition.

Les cheveux traités avec la composition (A) selon l'invention ont un toucher plus naturel, plus doux. La composition (A) ne poudre pas les cheveux après séchage et peignage. De plus le pouvoir laquant de la composition (A) est supérieur à celui de la composition (B).

### EXEMPLE 3

On a préparé une composition (C) de spray fixant conditionnée dans un récipient aérosol de composition suivante :
- Terpolymère acide acrylique / acrylate d'éthyle / n-tertio butylacrylamide vendu sous la dénomination ULTRAHOLD STRONG par la société BASF 6,1 g
- Amino-2 méthyl-2 propanol-1 qs neutralisation à 100% du polymère
- Silicone oxyalkylénée vendue à 10% de MA dans une cyclométhicone sous la dénomination FLUID DC 3225C par la société DOW CORNING 0,043 gMA
- Ethanol qsp 100 g

Schéma de pressurisation :

| | |
|---|---|
| Composition ci-dessus : | 53 g |
| Diméthyléther | 15 g |
| Butane | 32 g |

On introduit 53 g de la composition ci-dessus dans un récipient aérosol, on sertit une valve, puis on introduit le diméthyléther et le butane. La composition présente les mêmes propriétés que celles de l'exemple 1.

On a préparé de la même façon une composition (D) non conforme à l'invention de composition suivante :
- Terpolymère acide acrylique / acrylate d'éthyle / n-tertio butylacrylamide vendu sous la dénomination ULTRAHOLD STRONG par la société BASF 6,1 g
- Amino-2 méthyl-2 propanol-1 qs neutralisation à 100% du polymère
- Silicone oxyalkylénée vendue sous la dénomination FLUID DC 190 par la société DOW CORNING 0,043 gMA
- Cyclométhicone 0,387 g
- Ethanol qsp 100 g

Schéma de pressurisation :

| | |
|---|---|
| Composition ci-dessus : | 53 g |
| Diméthyléther | 15 g |
| Butane | 32 g |

On a appliqué chacune de ces compositions sur des cheveux lavés et séchés. Un panel de 5 testeurs expérimentés a évalué les propriétés de chaque composition.

Les cheveux traités avec la composition (C) selon l'invention ont un toucher plus naturel, plus doux que ceux traités avec la composition (D).

### EXEMPLE 4

On a préparé une composition de spray fixant conditionnée dans un récipient aérosol de composition suivante :
- Terpolymère acide acrylique / acrylate d'éthyle / n-tertio butylacrylamide vendu sous la dénomination ULTRAHOLD STRONG par la société BASF 7 g
- Amino-2 méthyl-2 propanol-1 qs neutralisation à 100% du polymère
- Silicone oxyalkylénée vendue sous la dénomination FLUID DC 3225C par la société DOW CORNING 0,03 gMA
- Eau 5 g
- Ethanol qsp 100 g

Schéma de pressurisation :

| | |
|---|---|
| Composition ci-dessus : | 45 g |
| Diméthyléther | 55 g |

On introduit 37 g de la composition ci-dessus dans un récipient aérosol, on sertit une valve, puis on introduit le diméthyléther et le pentane.

La composition présente les mêmes propriétés que celles de l'exemple 1.

## Revendications

1. Composition cosmétique, **caractérisée en ce qu'**elle comprend, dans un milieu cosmétiquement acceptable, au moins un polymère fixant anionique dérivé d'acide acrylique ou méthacrylique et au moins une silicone oxyalkylénée de formule (I) : 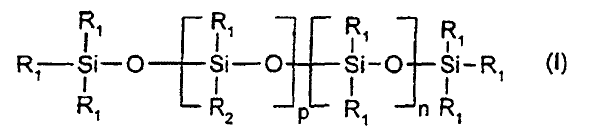 formule dans laquelle :
- R₁, identique ou différent, représente un atome d'hydrogène, un radical alkyle linéaire ou ramifié en C₁-C₃₀ ou un radical phényle,
- R₂, identique ou différent, représente -(CₓH₂ₓ)-(OC₂H₄)ₐ-(OC₃H₆)_{b}-OR₃
- R₃, identique ou différent, est choisi parmi un atome d'hydrogène, un radical alkyle, linéaire ou ramifié, ayant de 1 à 12 atomes de carbone, un radical acyle, linéaire ou ramifié ayant de 2 à 12 atomes de carbone,
- n varie de 100 à 1000,
- p varie de 1 à 8,
- a varie de 0 à 50,
- b varie de 0 à 50,
- a + b est supérieur ou égal à 1,
- x varie de 1 à 5,
le poids moléculaire moyen en nombre étant supérieur ou égal à 15.000.

2. Composition selon la revendication précédente, **caractérisée en ce que** les silicones oxyalkylénées de formule générale (I) répondent à au moins une des, et de préférence à toutes les, conditions suivantes :
- R₁ désigne le radical méthyle;
- R₃ représente un atome d'hydrogène, un radical méthyle ou un radical acétyle et de préférence hydrogène;
- p varie de 2 à 6;
- a est compris entre 5 et 40 et de préférence entre 15 et 30;
- b est compris entre 5 et 40 et de préférence entre 15 et 30;
- x est égal à 2 ou 3;
- n varie de 200 à 600 et de préférence de 300 à 500.

3. Composition selon l'une quelconque des revendications 1 ou 2, **caractérisée en ce que** le poids moléculaire moyen en nombre est compris entre 25.000 et 75.000.

4. Composition selon l'une quelconque des revendications précédentes, **caractérisée en ce que** le ou les polymères anionique fixants sont présents dans des concentrations comprises entre 0,1% et 20% en poids, et de préférence dans des concentrations comprises entre 1% et 10% en poids par rapport au poids total de la composition.

5. Composition selon l'une quelconque des revendications précédentes, **caractérisée en ce que** la ou les silicones oxyalkylénées sont présentes dans des concentrations comprises entre 0,001% et 10% en poids, et de préférence dans des concentrations comprises entre 0,005% et 3% en poids.

6. Composition selon l'une quelconques des revendications précédentes, **caractérisée en ce que** le polymère fixant anionique est choisi parmi :
A) les homopolymères d'acide acrylique ou méthacrylique ou leurs sels.
B) les copolymères d'au moins un acide acrylique et/ou méthacrylique avec au moins un monomère monoéthylénique tel que l'éthylène, le styrène, les esters vinyliques, les acrylamides ou méthacrylamides éventuellement N-substitués, les esters d'acide acrylique ou méthacrylique, les vinyllactames.

7. Composition selon l'une quelconque des revendications précédentes, **caractérisée par le fait que** le milieu cosmétiquement acceptable comprend au moins un alcool en C₁-C₆.

8. Composition selon l'une quelconque des revendications précédentes, **caractérisée par le fait qu'**elle se présente sous forme de gel, de lait, de crème, de dispersion, de lotion plus ou moins épaissie ou de mousse.

9. Composition selon l'une quelconque des revendications précédentes, **caractérisée par le fait qu'**elle est un produit de coiffage, de maintien de la coiffure ou de mise en forme.

10. Composition selon l'une quelconque des revendications précédentes, **caractérisée par le fait qu'**elle est conditionnée sous forme de vaporisateur, de flacon pompe ou bien dans un récipient aérosol en vue d'obtenir un spray, une laque ou une mousse.

11. Composition selon l'une quelconque des revendications précédentes, **caractérisée par le fait qu'**elle comprend un agent propulseur.

12. Composition selon la revendication précédente, **caractérisée par le fait qu'**elle comprend de 10 à 90 % en poids d'un agent propulseur par rapport au poids total de la composition.

13. Procédé de traitement cosmétique des matières kératiniques en particulier des cheveux, **caractérisée par le fait qu'**il consiste à appliquer sur lesdites matières une composition telle que définie selon l'une quelconque des revendications précédentes.

## Patentansprüche

1. Kosmetische Zusammensetzung, **dadurch gekennzeichnet, dass** sie in einem kosmetisch akzeptablen Medium mindestens ein anionisches fixierendes Polymer, das von Acrylsäure oder Methacrylsäure abgeleitet ist, und mindestens ein alkoxyliertes Silicon der Formel (I) enthält: 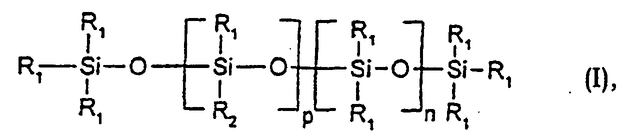 wobei in der Formel:
- die Gruppen R₁, die identisch oder voneinander verschieden sind, Wasserstoff, eine geradkettige oder verzweigte C₁₋₃₀-Alkylgruppe oder Phenyl bedeuten,
- die Gruppen R₂, die identisch oder voneinander verschieden sind, -(CₓH₂ₓ)-(OC₂H₄)ₐ-(OC₃H₆)_{b}-OR₃ bedeuten,
- die Gruppen R₃, die identisch oder voneinander verschieden sind, unter Wasserstoff, geradkettigen oder verzweigten Alkylgruppen mit 1 bis 12 Kohlenstoffatomen oder geradkettigen oder verzweigten Acylgruppen mit 2 bis 12 Kohlenstoffatomen ausgewählt sind,
- n im Bereich von 100 bis 1000 liegt,
- p im Bereich von 1 bis 8 liegt,
- a im Bereich von 0 bis 50 liegt,
- b im Bereich von 0 bis 50 liegt,
- a + b 1 bedeutet oder darüber liegt,
- x im Bereich von 1 bis 5 liegt,
- wobei das Zahlenmittel des Molekulargewichts mindestens 15.000 beträgt.

2. Zusammensetzung nach dem vorhergehenden Anspruch, **dadurch gekennzeichnet, dass** die alkoxylierten Silicone der allgemeinen Formel (I) mindestens eine und vorzugsweise alle folgenden Bedingungen erfüllen:
- R₁ bedeutet Methyl;
- R₃ bedeutet Wasserstoff, Methyl oder Acetyl und vorzugsweise Wasserstoff;
- p liegt im Bereich von 2 bis 6;
- a liegt im Bereich von 5 bis 40 und vorzugsweise 15 bis 30;
- b liegt im Bereich von 5 bis 40 und vorzugsweise 15 bis 30;
- x bedeutet 2 oder 3;
- n liegt im Bereich von 200 bis 600, vorzugsweise 300 bis 500.

3. Zusammensetzung nach einem der Ansprüche 1 oder 2, **dadurch gekennzeichnet, dass** das Zahlenmittel des Molekulargewichts im Bereich von 25.000 bis 75.000 liegt.

4. Zusammensetzung nach einem der vorhergehenden Ansprüche, **dadurch gekennzeichnet, dass** das oder die fixierenden anionischen Polymere in Konzentrationen von 0,1 bis 20 Gew.-% und vorzugsweise in Konzentrationen von 1 bis 10 Gew.-%, bezogen auf das Gesamtgewicht der Zusammensetzung, vorliegen.

5. Zusammensetzung nach einem der vorhergehenden Ansprüche, **dadurch gekennzeichnet, dass** das oder die alkoxylierten Silicone in Konzentrationen im Bereich von 0,001 bis 10 Gew.-% und vorzugsweise in Konzentrationen von 0,005 bis 3 Gew.-%, bezogen auf das Gesamtgewicht der Zusammensetzung, vorliegen.

6. Zusammensetzung nach einem der vorhergehenden Ansprüche, **dadurch gekennzeichnet, dass** das anionische fixierende Polymer ausgewählt ist unter:
A) Homopolymeren von Acrylsäure oder Methacrylsäure oder deren Salzen;
B) Copolymeren von zumindest Acrylsäure und/oder Methacrylsäure mit mindestens einem monoethylenisch ungesättigten Monomer, wie Ethylen, Styrol, Vinylestern, gegebenenfalls N-substituierten Acrylamiden oder Methacrylamiden, Estern von Acrylsäure oder Methacrylsäure und Vinyllactamen.

7. Zusammensetzung nach einem der vorhergehenden Ansprüche, **dadurch gekennzeichnet, dass** das kosmetisch akzeptable Medium mindestens einen C₁₋₆-Alkohol enthält.

8. Zusammensetzung nach einem der vorhergehenden Ansprüche, **dadurch gekennzeichnet, dass** sie als Gel, Milch, Creme, Dispersion, mehr oder weniger dickflüssige Lotion oder Schaum vorliegt.

9. Zusammensetzung nach einem der vorhergehenden Ansprüche, **dadurch gekennzeichnet, dass** es sich um ein Produkt zum Frisieren, zur Festigung der Frisur oder zur Formgebung handelt.

10. Zusammensetzung nach einem der vorhergehenden Ansprüche, **dadurch gekennzeichnet, dass** sie in einem Zerstäuber, Pumpflakon oder Aerosolbehälter konfektioniert ist, um ein Spray, einen Lack oder einen Schaum zu erhalten.

11. Zusammensetzung nach einem der vorhergehenden Ansprüche, **dadurch gekennzeichnet, dass** sie ein Treibmittel enthält.

12. Zusammensetzung nach dem vorhergehenden Anspruch, **dadurch gekennzeichnet, dass** sie 10 bis 90 Gew.-% eines Treibmittels, bezogen auf das Gesamtgewicht der Zusammensetzung, enthält.

13. Verfahren zur kosmetischen Behandlung von Keratinsubstanzen und insbesondere Haaren, **dadurch gekennzeichnet, dass** es darin besteht, auf die Keratinsubstanzen eine Zusammensetzung nach einem der vorhergehenden Ansprüche aufzutragen.

## Claims

1. Cosmetic composition, **characterized in that** it comprises, in a cosmetically acceptable medium, at least one anionic fixing polymer derived from acrylic or methacrylic acid and at least one oxyalkylenated silicone of formula (I): 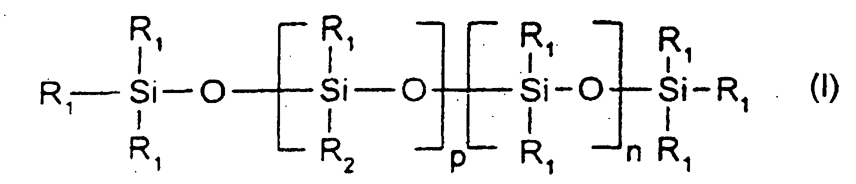 in which formula:
- R₁, which may be identical or different, represents a hydrogen atom, a linear or branched C₁-C₃₀alkyl radical or a phenyl radical,
- R₂, which may be identical or different, represents - (CₓH₂ₓ)-(OC₂H₄)ₐ-(OC₃H₆)_{b}-OR₃,
- R₃, which may be identical or different, is chosen from a hydrogen atom, a linear or branched alkyl radical having from 1 to 12 carbon atoms or a linear or branched acyl radical having from 2 to 12 carbon atoms,
- n ranges from 100 to 1000,
- p ranges from 1 to 8,
- aranges from 0 to 50,
- b ranges from 0 to 50,
- a + b is greater than or equal to 1,
- x ranges from 1 to 5, the number-average molecular weight being greater than or equal to 15,000.

2. Composition according to the preceding claim, **characterized in that** the oxyalkylenated silicones of general formula (I) satisfy at least one, and preferably all, of the following conditions:
- R₁ denotes a methyl radical;
- R₃ represents a hydrogen atom, a methyl radical or an acetyl radical and preferably hydrogen;
- p ranges from 2 to 6;
- a is between 5 and 40 and preferably between 15 and 30;
- b is between 5 and 40 and preferably between 15 and 30;
- x is equal to 2 or 3;
- n ranges from 200 to 600, preferably from 300 to 500.

3. Composition according to either of Claims 1 and 2, **characterized in that** the number-average molecular weight is between 25,000 and 75,000.

4. Composition according to any one of the preceding claims, **characterized in that** the fixing anionic polymer(s) is(are) present in concentrations of between 0.1% and 20% by weight, and preferably in concentrations of between 1% and 10% by weight,. relative to the total weight of the composition.

5. Composition according to any one of the preceding claims, **characterized in that** the oxyalkylenated silicone(s) is(are) present in concentrations of between 0.001% and 10% by weight, and preferably in concentrations of between 0.005% and 3% by weight.

6. Composition according to any one of the preceding claims, **characterized in that** the anionic fixing polymer is chosen from:
A) Homopolymers of acrylic or methacrylic acid or their salts;
B) Copolymers of at least one acrylic and/or methacrylic acid with at least one monoethylenic monomer such as ethylene, styrene, vinyl esters, optionally N-substituted acrylamides or methacrylamides, acrylic or methacrylic acid esters and vinyllactams.

7. Composition according to any one of the preceding claims, **characterized in that** the cosmetically acceptable medium comprises at least one C₁-C₆alcohol.

8. Composition according to any one of the preceding claims, **characterized in that** it is in the form of a gel, a milk, a cream, a dispersion, a more or less thickened lotion or a foam.

9. Composition according to any one of the preceding claims, **characterized in that** it is a styling product or a product for holding the hairstyle or for shaping the hair.

10. Composition according to any one of the preceding claims, **characterized in that** it is packaged in the form of a vaporizer, a pump-dispenser bottle or in an aerosol container in order to obtain a spray, a lacquer or a foam.

11. Composition according to any one of the preceding claims, **characterized in that** it comprises a propellant.

12. Composition according to the preceding claim, **characterized in that** it comprises from 10 to 90% by weight of a propellant relative to the total weight of the composition.

13. Cosmetic treatment process for keratin substances, in particular the hair, **characterized in that** it consists in applying to the said substances a composition as defined according to any one of the preceding claims.
